# EUROPEAN PATENT APPLICATION

(11) **EP 2 630 944 A1**
(43) Date of publication of application: **28.08.2013**
(21) Application number: 12156749.9
(22) Date of filing: 23.02.2012
(51) Int. Cl.: A61K 8/25, A61K 8/58, A61Q 11/00, A61K 8/02, A61Q 11/02, A61K 8/34, A61K 8/86

(54) **Silicon-based particle composition**

(71) Applicant: Herzog, Edmund, 87527 Sonthofen (DE); Wandel, Dominik, 87488 Betzigau (DE)
(72) Inventor: Herzog, Edmund, 87527 Sonthofen (DE); Wandel, Dominik, 87488 Betzigau (DE)
(74) Representative: Jansen, Cornelis Marinus

(57) **Abstract**

The present invention is directed to a composition comprising pharmaceutically acceptable silicon-based particles, at least one pharmaceutically acceptable dispersant, and optionally one or more binders or thinking agents, pharmaceutically acceptable surface active viscosity builders, flavour additives, preservatives and/or food colorants for use in the surface sealing of mammal teeth and/or dentures.

## Description

### TECHNICAL FIELD

The present invention relates to a composition and a pharmaceutically composition, respectively, comprising pharmaceutically acceptable silicon-based particles sealing the surface of teeth and/or dentures for use in the prevention and/or treatment of, for example, dental caries, periodentitis, dental hypersensitivity, neck-of-tooth sensitivity, secondary infections and/or tooth discoloration in a mammal. Preferably, the active agent is a silicon-based particle for use in the surface sealing, i.e. in the sealing of the capillary cavities in the surface of teeth and/or dentures.

The composition optionally comprises a minimum amount of excipients required to disperse the composition in a dosage form or apply it to a health care product, preferably the surface of such product, which then releases the silicon-based particles to the surface of teeth and/or dentures. The silicon-based particles are preferably in the form of association colloids. Furthermore, the composition optionally comprises active agents which support the prevention and/or treatment of, for example, dental caries, periodentitis, dental hypersensitivity, neck-of-tooth sensitivity, secondary infections and/or tooth discoloration.

The invention further relates to methods for the production of said composition for use in the prevention and/or treatment of dental caries, periodentitis, dental hypersensitivity, neck-of-tooth sensitivity, secondary infections and/or tooth discoloration.

### BACKGROUND OF THE INVENTION

The overwhelming necessity for effective and causal prevention of dental diseases, especially dental caries is well known in the art. Dental caries is an irreversible infection of usually bacterial origin that causes demineralization of the hard tissues (enamel, dentin and cementum) and leads to destruction of the organic matter of the tooth, usually by the influence of acid. Some of the bacterias who build and are part of this biofilm, are able to produce enough acid from metabolizing many kinds of carbohydrates, which come to them through the typical kinds of food mammals usually take. An indispensible prerequisite for the development of caries and most kinds of gingival inflammation is the presence of dental plaques on the dental surfaces. Worldwide, most children and an estimated 90% of adults have experienced caries, with the disease most prevalent in Latin American countries, countries in the Middle East, and South Asia, and least prevalent in China. In the United States, dental caries is the most common often chronic childhood disease, being at least five times more common than asthma. It is the primary pathological cause of tooth loss in children. In Germany, only 1% of all adults have never experienced caries. The forth German study on mouth hygiene (DMS IV; 2010) revealed that 29.9% of children below 12 years, 53.9% of adolescents below 15 years and 99% of all adults have suffered from caries. These figures illustrate the remaining strong demand for adequate prevention and treatment of caries, respectively.

Even though the number of cases has decreased in some developed countries, and this decline is usually attributed to increasingly better oral hygiene practices and preventive measures such as fluoride treatment, more measures are necessary in order to reduce individual pain and global economic loss.

The mouth contains a wide variety of oral bacteria, but only a few specific species of bacteria are believed to cause dental caries: Streptococcus mutans and Lactobacilli are among them. Lactobacillus acidophilus, Actinomyces viscosus, Nocardia spp., and Streptococcus mutans are most closely associated with caries, in particular root caries. Bacteria collect around the teeth and gums in a sticky, creamy-coloured mass called plaque or nowadays "biofilm", in particular "bacterial biofilm".

Such dental plaque is characterized as a biofilm, which is usually a pale white-yellow film that develops naturally on the teeth. In the art, a biofilm is known as an aggregate of microorganisms in which cells adhere to each other on a surface. The skilled person is aware that biofilms form on living or non-living surfaces and are prevalent in natural, industrial and hospital settings. The microbial cells growing in a biofilm are physiologically distinct from planktonic cells of the same organism, which, by contrast, are single-cells that may float or swim in a liquid medium.

Like any biofilm, dental plaque is formed by colonizing bacteria trying to attach themselves to a smooth surface of the tooth. Some sites collect plaque more commonly than others. Grooves on the occlusal surfaces of molar and premolar teeth provide microscopic retention sites for plaque bacteria, as do the approximal sites. Plaque also collect above or below the gingiva where it is referred to as supra- or sub-gingival plaque respectively. Bacteria in a person's mouth convert glucose, fructose, and most commonly sucrose (table sugar) into acids such as lactic acid through a glycolytic process called fermentation. If left in contact with the tooth, these acids cause demineralization, which is the dissolution of its mineral content. Fluoride toothpaste or dental varnish may aid remineralization. If demineralization continues over time, enough mineral content may be lost so that the soft organic material left behind disintegrates, forming a cavity or hole. The impact such sugars have on the progress of dental caries is called cariogenicity. Sucrose, although a bound glucose and fructose unit, is in fact more cariogenic than a mixture of equal parts of glucose and fructose. This is due to the bacteria utilising the energy in the saccharide bond between the glucose and fructose subunits. *S.mutans* adheres to the biofilm on the tooth by converting sucrose into an extremely adhesive substance called dextran polysaccharide by the enzyme dextransucranase.

Existing preventative measures besides classical, for example, teeth brushing and using of dental floss suffer from certain drawbacks. For example, complete removement of the biofilm through domestic measures like Brushing or Flossing is very hard to achieve (30-40% biofilm is left on average); furthermore, complete abstinence from sugar and sugar-derived products as substrate for bacterial growth is hardly possible, besides the fact, that this regarding carbohydrates are found in many different kind of foods and drinks. The fluoride treatment is sometimes disputed for a disproportional risk of side effects. Preventive measures like the invention of Xylit ("sugar-free products" or the sealing of scissures could not ban the high risk of biofilm-development and the following consequences like caries or inflammation of the gums (periodentitis).

Preventive eradication of harmful bacteria sometimes requires disproportional means with regard to disinfectants or even antibiotics and professional dental interventions. Moreover, ultrasound removal of biofilms constitute an unpleasant and sometimes even harmful for the tooth-surface dentist treatment.

Besides dental caries, periodentitis is a common oral disease characterized by an inflammation of the periodontium, i.e., the tissues that support the teeth. The prime cause for periodentitis is the accumulation of a mycotic and bacterial matrix at the gum line, called dental plaque. Prevention is mainly limited to brushing, flossing and the use of antiseptic mouthwashes. Hence, like for dental caries, there is a need for an improved prevention for periodentitis preventing the formation of such dental plaques, i.e., the adhesion of biofilms to the dental surface. Previously, EP0737470 proposed oral care compositions comprising colloidal anti plaque agents, which were coated with moieties to recognize bacterial structures, for example in a biofilm. These moieties comprised, for example, antibodies or antibody fragments. However, performing such coatings is expensive and time-consuming. In addition, antibodies and antibody fragments bound to colloid particles always bear the risk of allergic reactions, especially as the colloid may serve as hapten to potentiate allergic reactions. Hence, a composition directed against plaques without such disadvantages would be of great benefit.

Moreover, neck-of-teeth sensitivity is known in the art to be a result of overexposing the neck of teeth to environmental impacts such as temperature and mechanical stress. Efficient sealing the neck of teeth to such mechanical stress would therefore significantly help in reducing such burden.

In addition, dental discolouring and pigment or calculus based discolouring of dentures is a condition in dental health which is more putting an aesthetical rather than a medical burden on the affected individual. Common use bleaching methods which comprise silica particles as abrasives or professional methods impair various risks, such as chemical burns from gel bleaching, if a high-concentration oxidizing agent contacts unprotected tissues, which bleach or discolor mucous membranes, sensitive teeth, over-bleaching, known in the profession as "bleached effect", particularly with the intensive treatments, i.e., products that provide a large change in tooth color over a very short treatment period, e.g., 1 hour, pain if one has "sensitive teeth" caused by open dentinal tubules, risk of increased hot/cold sensitivity or even increased risk of tongue cancer. Thus, an alternative that prevents dental discoloring or at least prolongs the period until another bleaching seems required would likewise be a direct health benefit for the affected individual.

For dentures, the invention introduces an easy way, to reduce pigment or calculus deposits and to maintain a smooth surface. This can lead to a significant improvement concerning the longevity of such dentures of all kinds and materials.

Thus, the problem underlying the present invention is a tremendous need for compositions for use in improved prevention and/or treatment of various dental diseases.

The problem is solved by the present invention which relates to a composition comprising silicon-based particles, wherein the composition features several unexpected advantages. The adhesion and formation of biofilms on dental surfaces is inhibited by the composition of the present invention. At the same time, the composition is relatively easy to prepare as no coating processes of colloids need to be performed and the composition does not bear the risk of allergic or toxic reactions against any antibodies or antibody fragments or antibiotics. As further consequence from these advantages, pain and follow-up costs from the aforementioned dental illnesses are reduced by adequate preventative employment of the composition according to the present invention. Furthermore, less otherwise aggressive means need to be executed both in prevention and treatment of dental diseases. Especially antibiotics and/or aggressive abrasive agents might be saved. Moreover, a positive cosmetic effect is observed.

### BRIEF DESCRIPTION OF FIGURES

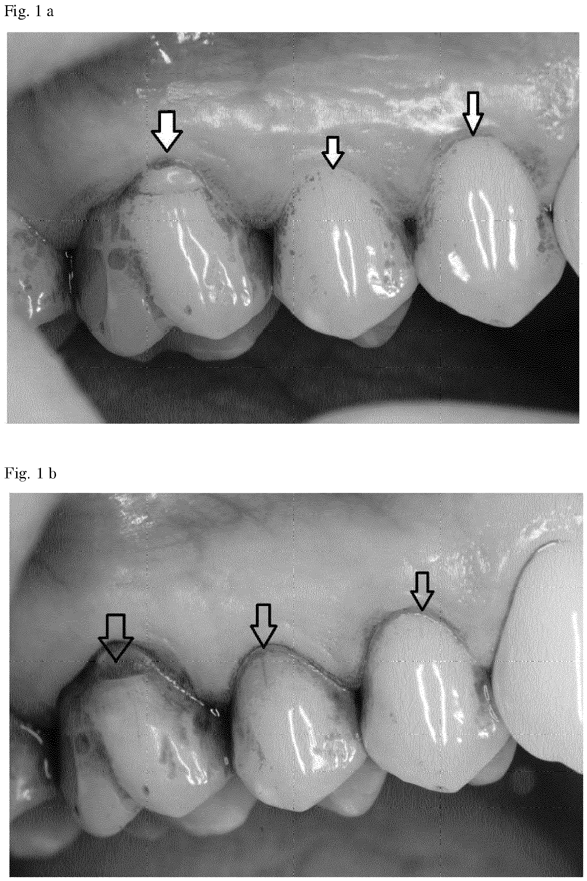

Fig. 1 a: Photograph of side cutters pre-treated with the claimed composition comprising silicon-based particles, dyed by a disclosing agent for biofilms after abstinence from teeth brushing for 48 h. Arrows indicate prominent dental surfaces only marginally affected by plaques.
Fig. 1 b: Photograph of side cutters of the same individual without pretreatment with the composition of the present invention comprising silicon-based particles (negative control), dyed by a disclosing agent for biofilms after abstinence from teeth brushing for 48 h. Solid arrows indicate prominent dental surfaces significantly affected by plaques in comparison to treatment.

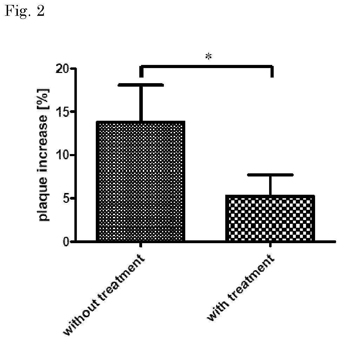

Fig. 2: Graph plotting the percental increase in biofilm coverage of side cutters with and without pre-treatment with the claimed composition comprising silicon-based particles over 48 h of abstinence from teeth brushing in comparison to biofilm coverage before abstinence from teeth brushing. * indicates significance (p-value < 0.05). Standard error of mean (SEM) is depicted in error bars.

### DETAILED DESCRIPTION

In the present invention, the inventors investigated a composition comprising silicon-based particles, for use in the prevention and/or treatment of dental diseases associated to the growth of microorganisms, especially bacteria, on the surface of teeth or dentures. The silicon-based particles are made from any type of silicon, preferably from mono-crystalline or monomolecular silicon, from silica or its derivatives. Alternatively, particles are made from siloxane, such as cyclopentasiloxane. In another alternative, the particles are formed from cyclopentasiloxane and mono-crystalline or monomolecular silicon, from silica and/or its derivatives. One preferred characteristic of cyclopentasiloxane is to increase surface density for example of a tooth and/or a denture.

In a preferred embodiment, the surface of the silicon particles is not modified for example by connection with an antibody, antigen or bioadhesive polymers. In another embodiment, the silicon particle has a surface activated by a chemical moiety, antibody, antigen or bioadhesive polymer. The particles are preferably association colloids formed from surface active silicon or siloxane such as cyclopentasiloxane. All such silicon-based particles are substantially water insoluble but exhibit surface activity by their large specific surface. Silicon in the sense of this application is understood as *"silicium",* not as a silicone polymer.

The composition preferably addresses capillary cavities and scissures in the surface of teeth hand/or dentures, further efficiently filling, hence, sealing them against intrusion by harmful microorganisms. Thus, the composition of the present invention is highly successful in the prevention and/or treatment of all processes detrimental to teeth or dentures or adjacent tissues initiated or sustained by harmful microorganisms, especially bacteria. For example, the composition is for use in the prevention and/or treatment of dental caries, periodentitis, secondary infections, neck-of-teeth sensitivity and/or tooth discoloration by inhibiting the formation of a biofilm and/or calculus and/or pigments on dental surfaces including all kinds of dentures. Preferred materials of dentures are metals and/or alloys for example for a tooth crown, body of a prosthesis, anchoring elements, complete anatomic crown; such metals are for example noble metals such as gold (high gold having > 75 % of gold, noble metal reduced having 60 to 75 % gold or platinum for example AU70Ag13.5Cu8.8 or Au70Pd15Pt7.5), palladium, alloys comprising silver, or fine gold (galvano technique, having > 85 % gold). Further, a denture consists of or comprises none noble metals such as cobalt (alloys of CoCr), nickel, ferrum, titan, preferably in the form of alloys, or pure titan for example for implants. All the alloys mentioned are suitable to be burned up or not. Moreover, a denture comprises or consists of ceramics for example as blinding material, scaffold material and/or anchoring elements. Such ceramics are for example blinding ceramics (e.g., compositions comprising feldspath such as K₂O•Al₂O₃•4SiO₂), oxide ceramics, (e.g., implants for ceramic of zircon oxide (ZrO₂), bridge- and crown scaffold), ceramic of aluminium oxide (Al₂O₃), or phosphate cement. Alternatively, a denture for example the body or base of prosthesis, filling material, blinding material, a prosthesis tooth consists or comprises for example PMMA (polymethylmethacrylate) or methacrylate and its derivatives, BisGMA (Bisphenol-A-(di)- methacrylate), Bis-EMA (ethoxylated bisphenol-A dimethacrylate), UDMA (urethandimethacrylate), TEGDMA (Triethylen-glycol-dimethacrylat), HEMA (Hydroxy-ethyl-methacrylat), or its derivatives, composite fillings (standard composition on w/w%):
Bis-GMA, UDMA, ethoxiliertes Bis-EMA (16.8)
Barium glass filler, ytterbium trifluoride, mied oxide (48.5)
Prepolymer (34)
Additives, catalysators and stabilisators (0.7)
Pigments (<0.1).

Dentures of the present invention comprise or consist of different combinations of the above mentioned materials.

The term biofilm is defined as complex structure adhering to surfaces that are regularly in contact with water, consisting of colonies of bacteria and optionally other microorganisms such as yeasts, fungi, and protozoa that secrete a mucilaginous protective coating in which they are encased. These bacteria and/or other microorganisms are for example often resistant to antibiotics and/or antimicrobial agents such as fungicides. Typical bacteria of such biofilms are for example Streptococcus mutans and Lactobacilli such as Lactobacillus acidophilus, Actinomyces viscosus, or Nocardia spp..

The composition preferably comprises pharmaceutically acceptable silicon.based particles, at least one pharmaceutically acceptable dispersant, and optionally one or more binders and thickening agents, pharmaceutically acceptable surface active viscosity builder, flavour additives and/or food colorants for use in the surface sealing of teeth and/or dentures. The composition of the invention is for use in mammals, for example a human, a domestic animal, in particular an animal for production.

All compositions of the invention are for use in cosmetic and/or medical prevention and/or treatment.

The particles are optionally characterized by their surface charge. The skilled person is aware that the zeta potential, which characterises electrokinetic potentials in colloidal systems, is the technical term to do so. The zeta potential is the potential difference between the dispersion medium and the stationary layer of fluid attached to the dispersed particle. A value of 25 mV (positive or negative) is generally recognized in the art as the value that separates low-charged surfaces from highly-charged surfaces. In the art, it is known to determine such value by dynamic light scattering.

Preferably, the silicon.based particles exhibit a zeta potential of 0 to -100 mV, preferably - 25 to - 70 mV, more preferably - 40 to -60 mV.

The silicon-based particles, preferably from mono-crystalline silicon, occupy the cavities and bind to the surface thanks to their high relative surface developing van der Waals attraction forces. These particles have a strong negative zeta potential of about -25 to -70 mV under physiological conditions, which increases the repulsion to dispersed particles of a likewise negative zeta potential. Bacteria responsible for dental caries are known in the art to have a zeta potential of about - 20 mV. Likewise, most dirt particles have a negative zeta potential. Thus, by repulsion and sterical hindrance, harmful microorganisms and dirt are prevented from entering the cavities in both teeth and dentures leading to a prevention and/or treatment of dental caries and associated microorganism-derived illnesses as well as to reduced dental discolouring.

Preferably, the teeth and/or dentures are sufficiently cleaned and/or disinfected, checked by the employment of respective disclosing agents, before sealing the surfaces with the composition of the present invention for the first time. Subsequently, the individual might employ a suitable dosage form comprising the composition him- or herself without the burden to seek professional help in the daily or regular application. Application might be applied on a monthly, weekly or daily basis depending on the individual needs. Application of the composition might be facilitated by administration of dosage forms for use in the oral cavity, but not for swallowing. In addition or alternatively, medicinal products or health care products might be coated with the composition, e.g. as a PEG-based polymer or paste, which subsequently release the composition to the teeth and/or dentures when used in the oral cavity or in an aqueous medium such as a dentures care liquid outside the oral cavity.

Further, the silicon.based particles are characterized by their mean particle size, which is likewise known in the art to be determined by dynamic light scattering. The particles of the present invention have a particle size of, for example, 0.1 to 1000 nm, preferably 20 to 800 nm, more preferably 50 to 600 nm, yet more preferably 100 to 400 nm, and most preferably 100 to 300 nm. Most preferred are sizes of for example 0.1, 10, 100, 250, 500, 750 or 1000 nm.

In the following, all percental indications refer to weight by weight percental rations (W/W), based on the total mass of the composition.

The composition is preferably a liquid, semi-solid or solid oral dosage form known to the person skilled in the art such as a mouthwash, toothpaste, chewing gum, gel, orodispersible tablet and lozenge, wherein the silicon-based particles are preferably dispersed in the oral dosage form.

The amount of silicon.based particles in such an oral dosage form is 0.01 to 10%, preferably 0.1 to 2% and more preferably 0.2 to 1% such as 0.375% in relation to the formulated oral dosage form, i.e. the above-described composition and further excipients to build a usable oral dosage form.

The oral dosage form, for example, comprises excipients necessary to build the oral dosage form. For example, in a liquid or semi-solid oral dosage form, the particles are dispersed in a pharmaceutically acceptable aqueous medium or diluent such as highly purified sterile water. Preferably, the medium has physiological osmolar strength and does not neutralize the zeta potential of the particles to collapse and to create aggregates. Hence, the medium is for example 0.9% NaCl or 25 nM 2-(4-(2-Hydroxyethyl)-1-piperazinyl)-ethansulfonic acid (HEPES) or at another concentration common in the art, or phosphate buffered saline (PBS), to prepare a dispersion of the claimed composition. As particles of the described size do not sediment, no suspension-stabilizing additives are required. However, a dispersant facilitates, for example, better dispersion properties of the particles in the dispersion and a viscosity builder facilitates better interaction of the dispersed particles with the target surface.

In a preferred embodiment, the diluent is highly purified water, glycerol 0.1 to 80%, preferably 0.1 to 50%, more preferably 1 to 20%, most preferably 1 to 5% such as 2%, or artificial salvia comprising potassium chloride, calcium chloride, sodium chloride and carboxymethyle cellulose preferably at 0.1 to 1%, 1 to 15%, 0.01 to 1% and 0.1 to 5%, respectively, most preferably 0.1, 1, 0.1 and 0.5%, respectively. The diluent is, for example, further pH-regulated, for example by a buffer substance such as anhydrous citric acid, preferably in an amount of 0.01 to 3%, preferably, 0.1 to 1%, most preferably 0.1%.

For example, tooth paste as semisolid composition comprises aluminum hydroxide (Al(OH)₃), calcium carbonate (CaCO₃), various calcium hydrogen phosphates, various zeolites, hydroxyapatite, baking soda, aloe, eucalyptus oil, myrrh, plant extract (such as strawberry extract), and essential oils.

In another embodiment, a chewing gum as a solid oral formulation comprises for example xylitol, sorbitol, other gum sweeteners or additives, such as polyols and carbamide.

In another embodiment of the present invention, the composition is a coating of a health care product, for example covering the surface of the health care product such as a tooth brush, interdental brush, dental floss, or an agent for cleaning and/or maintaining dentures, wherein the composition is optionally released from the surface of the health care product in physiological oral environment and wherein the amount of silicon-based particles is 1 to 50%, preferably 2 to 20% and more preferably 4 to 6%.

In addition, the composition comprises a dispersant in an amount of 0.01 to 60%, preferably 0.1 to 2% or 35 to 40%, and further optionally, the composition comprises the surface active viscosity builder in an amount of 0.01 to 20%, preferably 0.1 to 2% or 3 to 8%.

The dispersant is, for example, selected from the group consisting of propylene glycol, di-propylene glycol methylether (DMP), glyceryl triacetate, vinyl alcohol, neoagarobiose, sugar polyols, glycerol, sorbitol, xylitol, maltitol, polymeric polyols, polydextrose, quillaia and urea.

Additionally, the composition optionally comprises an amphoteric substance such as betaine.

In an alternative embodiment, the composition is conserved by a pharmaceutically acceptable preservative such as sorbic acid and its salts, benzoic acid and its salts, calcium propionate, disodium EDTA and/or benzalconium chloride. The preservative is, for example, present in an amount of 0.01 to 5%, preferably 0.01 to 0.1%. Further, in a preferred embodiment, the composition comprises colour agents such a s Patent Blue V or green agent E131, preferably in the amount of 0.01 to 1%, preferably 0.01 to 0.1% and/or flavour agents such as peppermint oil, lemmon oil and/or orange oil, preferred in the amount of 0.01 to 1%, more preferred in the amount of 0.1 to 0.5%.

In another embodiment, the composition comprises active agents which support in the prevention and/or treatment of dental disorders as described above by various means. These further active ingredients are either individually present in the composition structurally independent from the silicon-based particles, or they are, for example, associated to the silicon-based particles, whereby the silicon-based particles also function as carriers.

In one embodiment, the composition comprises for example antiphlogistic agents such as panthenol, e.g. in an amount of 1 to 10%, or hyaloronic acid, e.g. in an amount of 0.05 to 10%, preferably in an amount of 0.1 to 5%. The composition further optionally comprises adstringent agents such as aluminum chloride, aluminum formiate, tormentill, myrrh tincture and/or ratanhia tincture in an amount of 1 to 98%, preferably 1 to 10%, more preferably 1 to 5%, respectively.

The composition optionally further comprises an antiseptic agent such as povidon-iodine in an amount of 0,5 to 15%, preferably 0.5 to 1%, chlorhexidinegluconate in an amount of 0.01 to 5%, preferably 0.01 to 0.05%, benzyldiamin HCL in an amount of 0.01 to 5%, preferably 0.01 to 1%, cetylpyridinium chloride 0.01 to 5%, preferably 0.01 to 1%, dequalinium chloride in an amount of 0.01 to 5%, preferably 0.01 to 0.1%, hexidine in an amount of 0.01 to 20%, preferably 0.1 to 5%, and/or ethacridine lactate in an amount of 0.1 to 20%, preferably 0.1 to 1%.

In a further embodiment, the composition comprises a local anaesthetic agent such as lidocaine or benzocaine in an amount of 1 to 8%, preferably 1 to 2%, respectively.

In addition, the composition optionally comprises plant-derived antiphlogistic agents such as ginger tincture in an amount of 1 to 75%, preferably 20 to 50%, circulation-enhancing plant-derived agents such as piperin in an amount of 1 to 75%, preferably 1 to 2%, teeth-remineralizing agents such as amine fluoride, sodium fluoride in an amount of 0.001 to 5%, preferably 0.01 to 0.05%, respectively, calculus inhibitors such as pyrophosphates and/or polyphosphonates in an amount of 0.1 to 10%, preferably 0.1 to 1%, neck-of-teeth desensitising agents such as strontium chloride in an amount of 0.1 to 10%, preferably 0.1 to 1% and/or calcium in a pharmaceutically acceptable and applicable form to reinforce teeth remineralisation in an amount of 0.1 to 10%, preferably 0.1 to 1%.

The composition optionally further comprises a surface active viscosity builder which is for example selected from the group consisting of cocamidopropyl hydroxysultaine polyethylenglycol, gelatin and cocamidopropyl betaine. The cocamidopropyl betaine preferably is free of amidoamine and dimethylaminopropylamine to avoid side effects. The surface active viscosity builders contribute, for example, to mediate a desired interaction between the silicon-based particles of the composition and the target surface of teeth and/or dentures.

In yet another preferred embodiment of the invention, the composition is for use in preventing the adhesion of a biofilm by sealing capillary cavities on the tooth and/or dentures. Therein, the sealing of the teeth saves areas close to already negatively affected teeth and/or dentures from being likewise becoming negatively affected and therefore contribute to a treatment of dental diseases.

In still another preferred embodiment of said aspect the composition is for use in preventing and/or treating of dental caries, periodentitis, secondary infections and/or tooth discoloration.

The composition or the pharmaceutical composition is administered 1 time per day, 2 times per day, 3 times per day, 4 times per day, 5 times per day, 6 times per day, 1 time per week, 2 times per week, 3 times per week, 4 times per week, 5 times per week or 6 times per week, 3 times per months, 2 times per months or 1 time per month. Preferably, the composition is administered in a compliance-enhancing scheme, such as 1 time per week as oral dosage form. Most preferably, the composition is administered after each intense tooth brushing following 5 to 30 minutes or 1 to 3 hours after a food intake for example 3 x per day.

The present invention is further directed to a method for the production of the composition, comprising the steps of mechanochemical top-down milling of silicon wafers for 1 to 72 h or providing siloxane starting material, and optionally analysing particle size and/or surface zeta potential. Subsequently, the particles are mixing with a dispersant and/or dispersed in a medium and/or diluent, and optionally mixed with one or more binders and/or thinking agents, preservatives, buffer substance, flavour additives and/or food colorants for 1 to 72 h in a protective atmosphere.

As described above, growth of microorganisms on the teeth imposes a risk for dental caries, periodentitis, neck-of-tooth sensitivity, dental hypersensitivity, secondary infections and/or tooth discoloration. Growth of microorganisms on dentures imposes the risk that these microorganisms will spread to adjacent original teeth or surrounding tissue. Of special interest are the small cavities on the surface of the teeth or dentures, which exist inherently and increase under daily mechanical and temperature stress. By their nature, they exhibit capillary forces which draw liquid inside. As almost all liquid present in the mouth is contaminated with microorganisms such as fungi and bacteria, these microorganisms find shelter in these cavities. By capillary forces, nutrients such as solved sugars are likewise absorbed into the cavities providing for ideal and protected growth conditions for the microorganisms. Conventional teeth hygiene might not reach these shelters. Likewise, otherwise harmless dirt might be absorbed into the cavities, ultimately leading to dental discolouring which might not be removed by conventional dental care products.

Hence, the sealing of these cavities by the composition of the present invention provides a solution to the above indicated problems, by protecting unaffected teeth from ingrowth of microorganisms and from dirt and other debris present in the mouth.

The following examples describe the present invention in more detail, which should not be construed as in any way limiting the scope of the invention.

### EXAMPLES

### Example 1: Preparation of monocrystalline silicon particles

Preparation started from crystalline silicon, available as commercial wafers p+-doped, 400 ± 25 µm of thickness. Following a procedure known from Halimaoui, 1997 and Moretti et al. 2006, the partial dissolution of the wafers was obtained in a HF-ethanol aqueous solution. The electrolytic solution was prepared by mixing HF (39.5% wt.) and anhydrous ethanol in a 3:7 ratio by volume. The wafer and the solution were placed in a Teflon cell where a current density of 40 mA/cm² was applied for 1 h. A current density peak of 800 mA/cm² has been used to release the porous silicon membranes, which had a mean porosity of 45% and a mean thickness of 120 µm.

The subsequent milling was carried out in a FRITSCH Pulverisette 6 planetary ball mill equipped with 80 mL agate vial and grinding balls of 10 mm diameter. The movement of grinding balls inside the vial ensures continuous impact with the material. Millimetric pieces of porous silicon samples and of crystalline silicon, for comparison purposes, previously subjected to a coarse grinding in an agate mortar, were placed in the vial and then sealed by a clamp. Milling conditions comprised the milling time of 1 to 72 h, preferably 2 to 10 h and the ball-to powder weight ratio of 10:1. After the milling run, the resulting nanopowder was collected in aseptic and antistatic conditions.

### Example 2: Preparations of mouthwash oral dosage forms comprising silicon-based particles

As compositions for oral use, several mouthwash formulations were prepared. For example, 0.375 parts (weight by weight) of silicon-based particles are dispersed in 98.24 parts of highly purified sterile water under constant stirring at room temperature in a protected atmosphere. 0.285 parts of DPM, 0.045 parts of a 33% (W/W) aqueous betaine solution, 0.15 parts of sodium benzoate, 0.1 parts of anhydrous citric acid, 0.01 parts of Patent blue V, 0.1 parts of propylene glycol, 0.6 parts of polyethylene glycol - glycerol hydroxysterate and 0.1 parts of peppermint oil are subsequently added. The resulting dispersion is allowed to stir for 1 h before the dispersion is filtrated via a 0.45 µm filter with a low adsorption profile.

Alternatively, 1.125 parts (weight by weight) of silicon-based particles are dispersed in 67.21 parts of highly purified sterile water under constant stirring at room temperature in a protected atmosphere. 0.855 parts of DPM, 0.135 parts of a 33% (W/W) aqueous betaine solution, 30.0 parts of ginger tincture, 0.01 parts of Patent blue V, 0.1 parts of propylene glycol, 0.6 parts of polyethylene glycol - glycerol hydroxysterate and 0.1 parts of peppermint oil are subsequently added. The resulting dispersion is allowed to stir for 1 h before the dispersion is filtrated via a 0.45 µm filter with a low adsorption profile. Alternatively, 0.375 parts (weight by weight) of silicon-based particles are dispersed in 91.95 parts of highly purified sterile water under constant stirring at room temperature in a protected atmosphere. 5.0 parts of panthenol, 1.0 parts of hyaloronic acid, 0.285 parts of DPM, 0.045 parts of a 33% (W/W) aqueous betaine solution, 0.15 parts of sodium benzoate, 0.1 parts of anhydrous citric acid, 0.01 parts of green color agent E104, 0.1 parts of propylene glycol, 0.6 parts of polyethylene glycol - glycerol hydroxysterate and 0.1 parts of orange oil are subsequently added. The resulting dispersion is allowed to stir for 1 h before the dispersion is filtrated via a 0.45 µm filter with a low adsorption profile.

Alternatively, 2.0 parts (weight by weight) of cyclopentasiloxane-based particles are dispersed in 96.94 parts of highly purified sterile water under constant stirring at room temperature in a protected atmosphere. 0.15 parts of sodium benzoate, 0.1 parts of anhydrous citric acid, 0.01 parts of Patent blue V, 0.1 parts of propylene glycol, 0.6 parts of polyethylene glycol - glycerol hydroxysterate and 0.1 parts of peppermint oil are subsequently added. The resulting dispersion is allowed to stir for 1 h before the dispersion is filtrated via a 0.45 µm filter with a low adsorption profile.

Alternatively, 0.375 parts (weight by weight) of silicon-based particles are dispersed in 95.34 parts of highly purified sterile water under constant stirring at room temperature in a protected atmosphere. 0.285 parts of DPM, 0.045 parts of a 33% (W/W) aqueous betaine solution, 1.0 parts of cetylpyridinium chloride, 2.0 parts of lidocaine, 0.15 parts of sodium benzoate, 0.1 parts of anhydrous citric acid, 0.01 parts of Patent blue V, 0.6 parts of polyethylene glycol - glycerol hydroxysterate and 0.1 parts of lemon oil are subsequently added. The resulting dispersion is allowed to stir for 1 h before the dispersion is filtrated via a 0.45 µm filter with a low adsorption profile.

### Example 3: Preparation of a coating comprising silicon-based particles

5 parts of (weight by weight) of silicon-based particles are dispersed in 5 parts of propylene glycol and 5 parts of sorbitol. The resulting dispersion is further dispersed in a substrate mass of 40 parts of carboxyvinyle alcohol and 44.9 parts of
polyvinylpyrrolidone. 0.1 parts of peppermint oil are added as flavor agent. The resulting mass is evenly and thinly applied on the surface of a dental floss.

### Example 4: Determination of biofilm inhibition efficacy in vivo

In a preliminary study, probands were subjected to a mouthwash employment, wherein the aqueous mouthwash composition comprised 0.375% (W/W) silicon-based particles. Probands were divided in two groups, in which both probands got a professional cleaning of teeth to eliminate plaques, i.e. biofilms. The teeth were then dyed by an adequate disclosing agent, such as Brillant Blue (C.I. 42090) and/or Phloxine B (C.I. 45410), to reveal eventual biofilm occurrence. The diagnostic finding was documented photographically, whereby dyed plaque surface was calculated by respective software (Photoshop CS 5.1 extended, Adobe). Upper side cutters were selected as site of interest to be investigated in the following.

Therefore, probands either received treatment in form of a mouthwash according to the present invention or did not receive any subsequent treatment. Next, abstinence for 48 h of teeth brushing was imposed, and biofilm growth was not interfered in the meantime. After abstinence period, teeth were dyed again by the same agent, and a photograph was taken from exactly the same spot of the teeth, i.e. the upper side cutters. Staining of the teeth either after abstinence with pre-treatment (Fig. 1 a) or without such pre-treatment (Fig. 1 b) turned out to be optically different.

Quantitative evaluation of the plaque formation on the teeth surface (see Table 1) revealed that pre-treatment resulted in a decrease in staining, i.e., in plaque and biofilm coverage. Fig. 2 shows the significant difference in biofilm coverage increase over the monitored period, the significance being determined by a paired t-test to beas low as p = 0.03.

**Table 1**

| **Proband** | **Increase in staining [%] w/o treatment** | **Increase in staining [%] with treatment** | **Δ** | **Relative decrease [%] by treatment** |
|---|---|---|---|---|
| 01 1.Q | 17,47 | 11,58 | 5,89 | 33,7 |
| 01 3.Q | 4,72 | 3,3 | 1,42 | 30,09 |
| 03 2.Q | 20,29 | 5,29 | 15 | 73,93 |
| 03 4.Q | 3,1 | -3,05 | 6,15 | 198,39 |
| 02 1.Q | 23,82 | 8,97 | 14,85 | 62,34 |

### Example 5: Determination of tolerability in vivo

Individuals who enrolled in the preliminary efficacy study were subjected to adverse effect monitoring. Therefore, a survey of possible adverse effects was elaborated and implemented for assessment before and after the treatment by the claimed composition comprising silicon-based particles. Mucosa burning, taste irritations, mucosa redness, mucosa bleeding, teeth- sensitivity, teeth discolouring, itching, generalized pain and generalized symptoms of an allergic reaction where monitored. No individual showed any adverse effect registered during enrolment.

## Claims

1. A composition comprising pharmaceutically acceptable silicon-based particles, at least one pharmaceutically acceptable dispersant, and optionally one or more binders or thinking agents, pharmaceutically acceptable surface active viscosity builders, flavour additives, preservatives and/or food colorants for use in the surface sealing of mammal teeth and/or dentures.

2. The composition according to claim 1, wherein the silicon-based particles are selected from the group consisting of monocrystalline silicon particles, monomolecular silicon particles and siloxane particles.

3. The composition according to any of claims 1 or 2, wherein the composition is formulated as an oral dosage form selected from the group consisting of mouthwash, toothpaste, chewing gum, orodispersible tablet and lozenge.

4. The composition according to any of claims 1 to 3, wherein the amount of silicon based particles is 0.01 to 10% (W/W), preferably 0.1 to 2% (W/W) and more preferably 0.2 to 1% (W/W).

5. The composition according to any of claims 1 or 2, wherein the composition is a coating of a health care product covering the surface of the health care product preferably selected from the group consisting of tooth brush, interdental brush, dental floss, or an agent for cleaning and/or maintaining dentures, wherein the composition is optionally released from the surface of the health care product.

6. The composition according to any of claim 5, wherein the amount of silicon based particles is 1 to 50% (W/W), preferably 2 to 20% (W/W) and more preferably 4 to 6% (W/W).

7. The composition according to claims 1 to 6, wherein the dispersant is selected from the group consisting of propylene glycol, di-propylene glycol methylether, glyceryl triacetate, vinyl alcohol, neoagarobiose, sugar polyols, glycerol, sorbitol, xylitol, maltitol, polymeric polyols, polydextrose, quillaia and urea.

8. The composition according to any of claims 1 to 7, wherein the surface active viscosity builder is selected from the group consisting of cocamidopropyl hydroxysultaine polyethylenglycol, gelatin and cocamidopropyl betaine.

9. The composition according to any of claims 1 to 8, wherein the silicon-based particles exhibit a zeta potential of 0 to -100 mV, preferably of - 25 to - 70 mV, more preferably of - 40 to -60 mV and a particle size of 10 to 500 nm, preferably of 30 to 350 nm, more preferably of 100 to 300 nm.

10. The composition according to any of claims 1 to 9, comprising the dispersant in an amount of 10 to 60% (W/W), preferably in an amount of 20 to 50% (W/W) and more preferably in an amount of 35 to 40% (W/W), and/or the surface active viscosity builder in an amount of 1 to 20% (W/W), preferably in an amount of 2 to 15% (W/W) and more preferably in an amount of 3 to 8% (W/W).

11. The composition according to any of claims 1 to 10, wherein the composition comprises further active agents which support in the prevention and/or treatment of dental disorders selected from the group consisting of antiphlogistic agents, adstingent agents, antiseptic agent, local anaesthetic agents, plant-derived antiphlogistic agents and/or teeth-remineralizing agents

12. The composition according to any of claims 1 to 11, wherein the composition is for use in preventing the adhesion of a biofilm by sealing capillary cavities on the tooth and/or dentures.

13. The composition according to any of claims 1 to 12, wherein the composition is for use in preventing and/or treating of dental caries, periodentitis, dental hypersensitivity, neck-of-tooth sensitivity, secondary infections and/or tooth discoloration.

14. The composition of claim 1 to 13, wherein the composition is administered 1 time per day, 2 times per day, 3 times per day, 4 times per day, 5 times per day, 6 times per day, 1 time per week, 2 times per week, 3 times per week, 4 times per week, 5 times per week or 6 times per week, 3 times per months, 2 times per months or 1 time per month.
